# EUROPEAN PATENT APPLICATION

(11) **EP 3 988 539 A1**
(43) Date of publication of application: **27.04.2022**
(21) Application number: 20798528.4
(22) Date of filing: 05.03.2020
(51) Int. Cl.: C07C 311/51, A61K 31/18, A61K 9/14, A61K 47/26, A61K 47/38, A61K 47/06, A61P 31/18

(54) **ACTIVE INGREDIENT, PHARMACEUTICAL COMPOSITION, AND MEDICINAL PREPARATION FOR THERAPY OF HIV AND AIDS**

(30) Priority: 30.04.2019 RU 2019113338
(71) Applicant: Alla Chem, LLC, Hallandale Beach, FL 33009 (US); Savchuk, Nikolay Filippovich, Rancho Santa Fe, CA 92067 (US); Ivashchenko, Andrey Alexandrovich, Moscow 127576 (RU); Ivachtchenko, Alena Alexandrovna, Hallandale, Florida 33009 (US); Ivachtchenko, Alexandre Vasilievich, Hallandale, FL 33009 (US)
(72) Inventor: IVACHTCHENKO, Alexandre Vasilievich, Hallandale, FL 33009 (US); SAVCHUK, Nikolay Filippovich, Rancho Santa Fe California, 92067 (US); IVASHCHENKO, Andrey Alexandrovich, Moscow, 127576 (RU); IVACHTCHENKO, Alena Alexandrovna, Hallandale, FL 33009 (US)
(74) Representative: Zellentin & Partner mbB Patentanwälte
(86) International application number: PCT/RU2020/000117
(87) International publication number: WO 2020/222675

(57) **Abstract**

This invention relates to the active ingredient of a pharmaceutical composition consisting of micronized monoclinic symmetry crystals (MMSCs) of sodium {[4-({[4-bromo-3-(3-chloro-5-cyanophenoxy)-2-fluorophenyl] acetyl }amino)-3-chlorophenyl] - sulfonyl}(propanoyl)azanide with an average particle diameter ≤130 µm and methods of use thereof in the treatment of Human Immunodeficiency Virus (HIV) and Acquired Immune Deficiency Syndrome (AIDS).

## Description

### FIELD OF THE INVENTION

This invention relates to the active ingredient of a pharmaceutical composition consisting of micronized monoclinic symmetry crystals of sodium {[4-({[4-bromo-3-(3-chloro-5-cyanophenoxy)-2-fluorophenyl] acetyl}amino)-3-chlorophenyl] - sulfonyl}(propanoyl)azanide with an average particle diameter ≤130 µm and methods of use thereof in the treatment of Human Immunodeficiency Virus (HIV) and Acquired Immune Deficiency Syndrome (AIDS).

### BACKGROUND OF THE INVENTION

The HIV virus infects the cells of the immune system that have CD4 receptors on their surface: T helpers, monocytes, macrophages, Langerhans cells, dendritic cells, microglial cells. This leads to immunodepression and development of AIDS; loss of patients' ability to protect themselves against infections and tumors; emergence of secondary opportunistic diseases that are not typical for people with normal immune status. Without medical intervention, HIV patients live an average of 9 to 11 years after infection (depending on the subtype of the virus) [https://ru.wikipedia.org/wiki/Virus_immunodefitsita_cheloveka].

According to the worldwide statistics [http://www.lenoblspid.ru/news24/postid/own_news/1166], in 2015, 36.7 million people globally were living with HIV, 2.1 million people were newly infected with HIV, and 1.1 million people died of AIDS-related illnesses. Since the start of the epidemic, 78 million people have become infected with HIV, of which 35 million people have died of AIDS-related illnesses.

As of December 2015, 17 million people living with HIV were receiving antiretroviral therapy, while in June 2015 this number was 15.8 million people and in 2010, 7.5 million people. In 2015, 46% of all adults living with HIV had access to treatment, whereas in 2010, only 23%.

Since 2010, new HIV infections have declined by 6%. In 2015, the global number of HIV-infected was 2.1 million people, while in 2010, 2.2 million people.

As compared to the highest figure in 2005, the rate of AIDS-associated mortality has declined by 45%, and in 2015, the number of individuals died due to AIDS worldwide was 1.1 million people against 2 million people in 2005.

HIV can be suppressed by combination antiretroviral therapy (ART) involving three or more antiretroviral drugs. ART does not cure HIV infection but suppresses viral replication within a person's body and allows the individual's immune system to strengthen and regain the capacity to fight off infections. Life expectancy for patients receiving ART may be extended to 70-80 years [http://www.who.int/mediacentre/factsheets/fs3 60/ru/].

In recent years, non-nucleoside reverse transcriptase inhibitors (NNRTIs) have attracted considerable attention from medical chemists and pharmacologists as ART drugs [Zhan P. et al. HIV-1 NNRTIs: structural diversity, pharmacophore similarity, and implications for drug design. Med. Res. Rev. 2013 Jun; 33 Suppl 1: E1-72. doi: 10.1002/med.20241. Epub 2011 Apr 26.].

There are known NNRTIs and pharmaceutical compositions that include these NNRTIs. In particular, the sodium salt 2-[4-bromo-3-(3-chloro-5-cyanophenoxy)-2-fluorophenyl]-N-(2-chloro-4-propionylsulfamoylphenyl)-acetamide (Ib) and 9 polymorphic crystalline forms thereof, as well as pharmaceutical compositions based thereon are known to be used for the treatment or prevention of HIV-mediated diseases [EA 019340 (2014)].

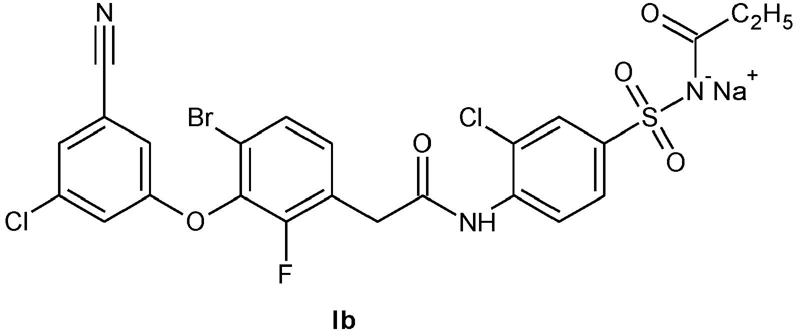

Patent EA 019340 provides examples of pharmaceutical compositions that include as an active ingredient any of the 9 crystalline polymorphic forms of the compound of formula Ib in a mixture with at least one pharmacologically acceptable carrier, diluent or inert filler. However, the patent examples do not specify which of the polymorphic crystal forms of the compound of formula Ib is used as the active ingredient and there is no data on the properties and effectiveness of the above compositions and finished dosage forms (FDFs).

### SUMMARY OF THE INVENTION

The subject of this invention is an active ingredient of pharmaceutical compositions in the form of micronized monoclinic symmetry crystals (MMSCs) of sodium {[4-(4-bromo-3-(3-chloro-5-cyanophenoxy)-2-fluorophenyl]acetyl}amino)-3-chlorophenyl]-sulfonyl}(propanoyl)azanide of formula Ib with an average particle diameter ≤130 µm and methods of application thereof in the treatment of human immunodeficiency virus (HIV) and acquired immune deficiency syndrome (AIDS).

Listed below are definitions of terms used to describe this invention.

The term "MMSCs" is an acronym for "micronized monoclinic symmetry crystals".

The term "micronized crystals" refers to crystals including crystals of organic substances with a micron average particle diameter.

The term "monoclinic symmetry crystals" refers to crystals whose unit cell is constructed on three vectors a, b, and c, which have different lengths, with two right angles and one indirect angle between them. Thus, the shape of the cell is determined by four parameters: the lengths of the base vectors a, b, and c, and the angle β, which differs from the right angle. The volume of the cell is equal to the product *a*·*b*·*c*-sinβ.

The term "pharmaceutical composition" refers to a composition comprising active ingredients and optionally at least one component selected from the group consisting of pharmaceutically acceptable and pharmacologically compatible inert fillers, solvents, diluents, carriers, excipients, and dispensers, delivery agents such as preservatives, stabilizers, fillers, disintegrators, moisteners, emulsifiers, suspending agents, thickeners, sweeteners, flavoring agents, aromatizing agents, antibacterial agents, fungicides, lubricants, and prolonged delivery controllers, the choice and proportions of which depend on the nature and route of administration and dosage. Examples of suitable suspending agents are ethoxylated isostearyl alcohol, polyoxyethylene, sorbitol and sorbitol ether, microcrystalline cellulose, aluminum metahydroxide, bentonite, agar-agar and tragacant, and mixtures thereof. Protection against microorganisms can be provided using various antibacterial and antifungal agents, such as parabens, chlorobutanol, sorbic acid, and the like. Said composition may also include isotonic agents, such as sugar, sodium chloride, and the like. The sustained action of the composition can be achieved using agents that decelerate the absorption of the active ingredient, for example, aluminum monostearate and gelatin. Examples of suitable carriers, solvents, diluents, and delivery agents include water, ethanol, polyalcohols, and mixtures thereof, natural oils (such as olive oil), and organic esters (such as ethyl oleate) for injections. Examples of fillers are lactose, milk sugar, sodium citrate, calcium carbonate, calcium phosphate, and the like. Examples of disintegrators and dispensers are starch, alginic acid and salts thereof, and silicates. Examples of lubricants are magnesium stearate, sodium lauryl sulfate, talc, and polyethylene glycol of high molecular weight.

The antiviral pharmaceutical composition can be in the form of a wide variety of orally administered dosage forms and carriers, including ones in the form of tablets, coated tablets, hard and soft gelatin capsules, solutions, emulsions, syrup or suspension. The antiviral pharmaceutical composition of the present invention is effective when administered as a suppository. The most convenient method of administration is usually oral, using the usual daily dosage regimen, which can be adjusted depending on the severity of the disease and the patient's response to the antiviral or antitumor medication.

An antiviral pharmaceutical composition with one or more conventional excipients, carriers, or diluents can be presented in the form of pharmaceutical compositions and single doses thereof. Pharmaceutical compositions and standard dosage forms may consist of conventional ingredients in conventional proportions with or without additional active compounds and dosage forms. The antiviral composition may contain any appropriate effective amount of the active ingredient commensurate with the prescribed daily dose. The antiviral composition can be used in the form of solid substances such as tablets or filled capsules, in the form of semi-solid powders, slow-release medications, or liquids, such as suspensions, emulsions, or filled capsules for oral administration; or in the form of suppositories for rectal or vaginal administration. A typical drug will contain about from 5% by weight up to 95% weight of the active compound or compound. The term "medication" or "dosage form" is intended to include both solid and liquid compositions of the active compound, and it will be clear to the person skilled in the art that the active ingredient may exist in the form of various medications, depending on the required dose and pharmacokinetic parameters.

Medications in solid form include powders, tablets, pills, capsules, suppositories, and dispersible granules. The solid carrier may be one or more substances that may also act as diluents, corrigents, solubilizers, lubricants, suspending agents, binders, preservatives, tablet disintegrating agents, or encapsulating material. In powders, the carrier is usually a finely ground solid, which is a mixture with a finely ground active ingredient. In tablets, the active ingredient is usually mixed with a carrier having the necessary binding capacity in suitable proportions and compressed into the desired shape of the desired size. Suitable carriers include, but are not limited to, magnesium carbonate, magnesium stearate, talc, sugar, lactose, pectin, dextrin, starch, gelatin, tragacanth, methylcellulose, sodium carboxymethylcellulose, fusible wax, cocoa butter, and the like. Medications in solid form may contain, in addition to the active ingredient, dyes, flavors, stabilizers, buffers, artificial and natural sweeteners, dispersants, thickeners, solubilizing agents, and the like.

Liquid formulations are also suitable for oral administration. Liquid dosage forms are emulsions, syrups, elixirs, and aqueous suspensions. These include solid forms of drugs that are designed to be converted into liquid drugs immediately before use. Emulsions can be prepared in solutions, for example, in aqueous solutions of propylene glycol, or may contain emulsifiers, such as lecithin, sorbitol monooleate, or gum arabic. Aqueous suspensions can be prepared by dispersing a finely ground active ingredient in water with viscous materials such as natural or synthetic gums, resins, methylcellulose, sodium carboxymethylcellulose, and other well-known suspending agents.

The antiviral pharmaceutical composition can be prepared for administration in the form of suppositories. Low-melting wax, such as a mixture of fatty acid glycerides or cocoa butter, is first melted and the active ingredient is homogeneously dispersed, for example, by stirring. The molten homogeneous mixture is then poured into molds of a convenient size and allowed to cool and harden.

The antiviral pharmaceutical composition can be prepared for vaginal administration using suppositories, tampons, creams, gels, pastes, foams, or sprays containing, in addition to the active ingredient, carriers commonly known in the field.

The subject of this invention is the active ingredient of pharmaceutical compositions, said ingredient being micronized monoclinic symmetry crystals (MMSCs) of sodium {[4-({[4-bromo-3-(3-chloro-5-cyanophenoxy)-2-fluorophenyl]acetyl}amino)-3-chlorophenyl]-sulfonyl}(propanoyl)azanide of formula Ib with an average particle diameter ≤130 µm.

The present invention also relates to an antiviral pharmaceutical composition containing, as an active ingredient, MMSCs of formula Ib with an average particle diameter ≤130 µm in combination with pharmacologically acceptable excipients.

Preferred is a pharmaceutical composition wherein monoclinic symmetry crystals of compound 1b are used, said crystals belonging to the monoclinic space group P21/c with unit cell parameters *a* = 15.0637(4)Å, *b* = 25.2592(6)Å, *c* = 6.89727(19)Å, β = 91.216(2)°, volume 2623.79(12) Å³, and melting point ~250°C.

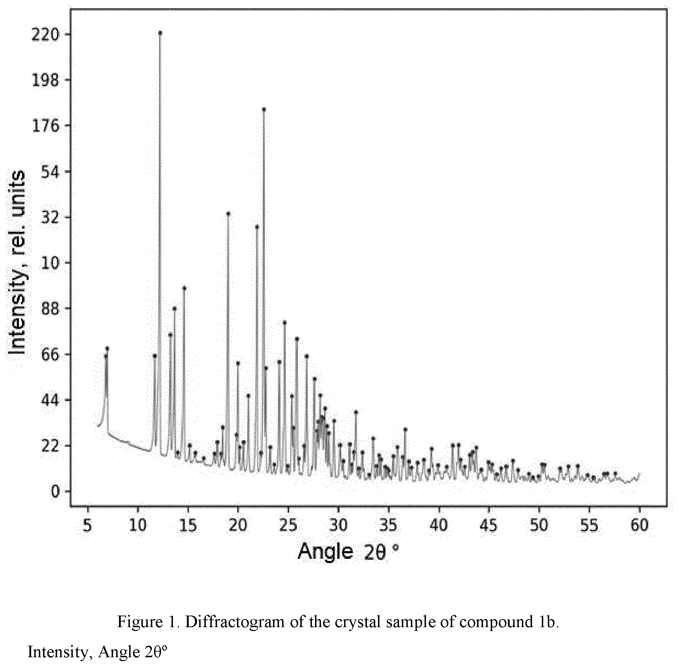

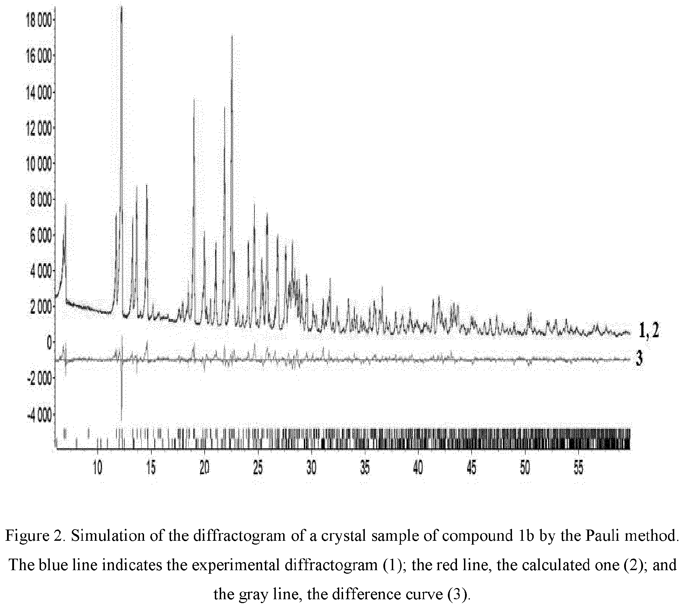

The characteristics of the crystalline samples of compound Ib were obtained from X-ray phase studies. The diffractograms of the samples show peaks whose positions correspond to a single monoclinic phase (Figs. 1 and 2, Table 1).

**Table 1. Interplanar distances (D) and relative intensities (Iᵣₑₗ= I/Iₒ×100) of the diffraction maxima of the sample of monoclinic phase 1b in the area of angles 2θ 6°-30°.**

| 2θ, ° | D, Å | Iᵣₑₗ, % |
|---|---|---|
| 6.8 | 13.0 | 18.0 |
| 7.0 | 12.7 | 22.0 |
| 11.7 | 7.6 | 29.0 |
| 12.2 | 7.2 | 100.0 |
| 13.3 | 6.7 | 29.0 |
| 13.7 | 6.5 | 37.0 |
| 14.6 | 6.1 | 39.0 |
| 18.5 | 4.8 | 10.0 |
| 19.0 | 4.7 | 67.0 |
| 20.0 | 4.4 | 26.0 |
| 20.6 | 4.3 | 7.0 |
| 21.1 | 4.2 | 26.0 |
| 21.9 | 4.1 | 70.0 |
| 22.5 | 3.9 | 85.0 |
| 22.8 | 3.9 | 21.0 |
| 24.1 | 3.7 | 28.0 |
| 24.7 | 3.6 | 39.0 |
| 25.4 | 3.5 | 22.0 |
| 25.9 | 3.4 | 30.0 |
| 26.6 | 3.4 | 8.0 |
| 26.8 | 3.3 | 28.0 |
| 27.6 | 3.2 | 24.0 |
| 28.0 | 3.2 | 15.0 |
| 28.2 | 3.2 | 25.0 |
| 28.4 | 3.1 | 13.0 |
| 28.7 | 3.1 | 14.0 |
| 28.9 | 3.1 | 15.0 |
| 29.1 | 3.1 | 11.0 |
| 29.6 | 3.0 | 18.0 |

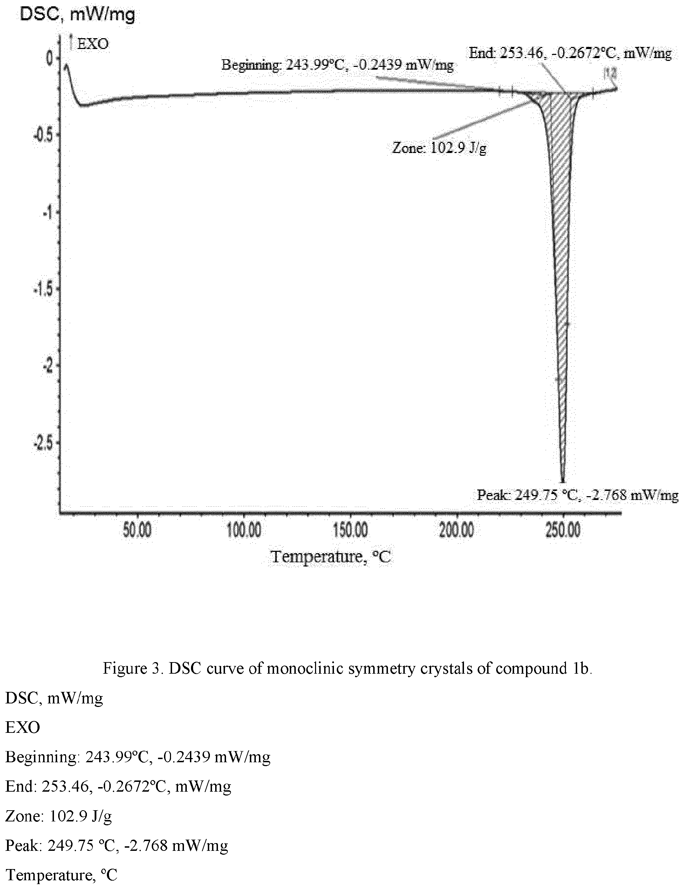

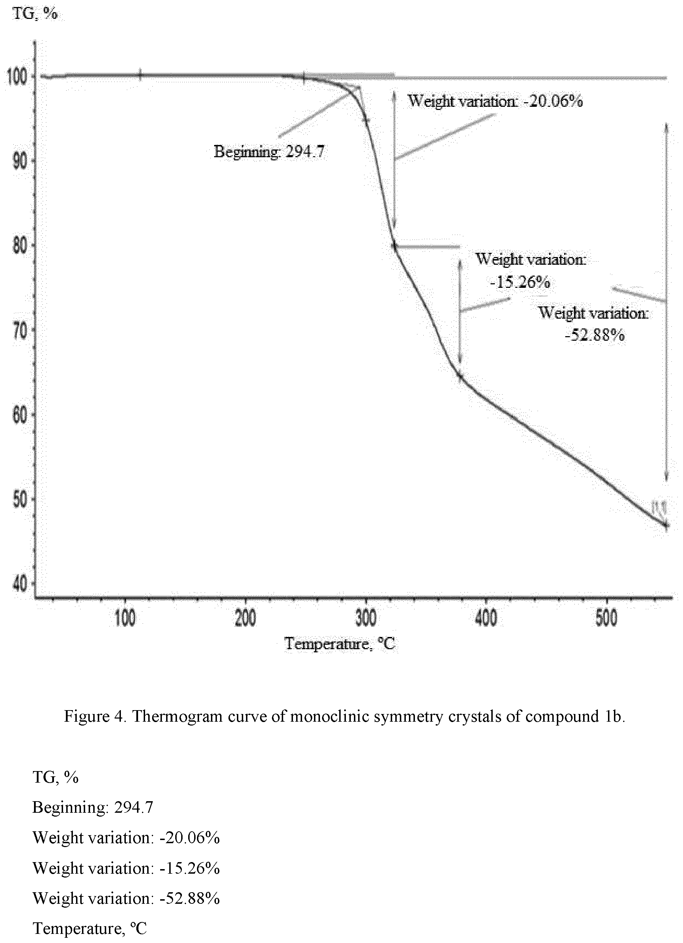

The melting point of monoclinic symmetry crystals of formula 1b (mp = ~250°C) was found using differential scanning calorimetry (DSC), with melting onset ~244°C, melting end ~253.5°C, and melting heat effect 102.9 J/g (Figure 3). The decomposition temperature of this product (found by thermogravimetric analysis) is 294.7°C (Figure 4).

Also preferred is a pharmaceutical composition wherein MMSCs of formula 1b with a particle diameter varying from 10 µm to 130 µm are used as the active ingredient and at least lactose monohydrate, sodium croscarmellose, polyvinylpyrrolidone (povidone), and magnesium stearate are used as excipients.

More preferred is a pharmaceutical composition containing 10.0% MMSCs of formula Ib, 81.7% lactose monohydrate, 5.1% croscarmellose sodium, 2.4% povidone, and 0.8% magnesium stearate.

Further preferred is a pharmaceutical composition containing 15.1% MMSCs of formula Ib, 76.2% lactose monohydrate, 5.4% croscarmellose sodium, 2.5% povidone, and 0.8% magnesium stearate.

The subject of the invention is a new medicinal product, which is a solid oral dosage form containing 20 mg, 40 mg, or 80 mg MMSCs of formula Ib with an average particle diameter of 10 to 130 µm in combination with excipients.

The subject of the invention is a novel medicinal product consisting of a capsule containing 20 mg, 40 mg, or 80 mg MMSCs of formula Ib with an average particle diameter of 10 to 130 µm in combination with lactose monohydrate, croscarmellose sodium, povidone and magnesium stearate.

More preferable is a medication in the form of a solid gelatin capsule No. 1 with a white body and a red cap, containing 20.7 mg or 40 mg of MMSCs of formula Ib with an average particle diameter of 10 to 130 µm in combination with 337.196 mg of lactose monohydrate, 21.168 mg of croscarmellose sodium, 9,800 mg of povidone, and 3.136 mg of magnesium stearate.

Another embodiment of the invention is a medication consisting of a solid gelatin capsule No. 4 with a white body and a red cap containing 20.7 mg or 40 mg of MMSCs of formula Ib with an average particle diameter of 10 to 130 µm in combination with 104.380 mg of lactose monohydrate, 7.390 mg of croscarmellose sodium, 3.430 mg of povidone and 1.100 mg of magnesium stearate.

The authors surprisingly found that the use in the pharmaceutical composition and FDF (finished dosage form) with the MMSCs of formula Ib wherein the average particle diameter is from 10 to 130 µm leads to a significant improvement in the pharmacological properties of these drugs. In particular, the use of the monoclinic form 1b in FDF and micronized granulation thereof to an average particle diameter of up to ≤130 µm leads to a noticeable improvement in the pharmacological parameters of FDF (Table 2) when administered to dogs.

**Table 2. Some pharmacological parameters of FDF following oral administration of 1 capsule containing 20.7 mg of MMSCs of formula Ib with an average particle diameter of 100 µm, 130 µm, or 200 µm at a dose of 1.4-1.6 mg/kg, depending on the dog's weight**

| | | | |
|---|---|---|---|
| Average particle diameter of MMSCs of formula Ib, µm | 200 | 130 | 100 |
| AUCₗₐₛₜ, h^{∗}ng/ml | 92.3 | 106.3 | 173.8 |
| Cₘₐₓ, ng/ml | 12.2 | 26.1 | 64.4 |

| | | | |
|---|---|---|---|
| **AUC** is a pharmacokinetic parameter that characterizes the total concentration of the drug in the blood plasma during the entire observation period. **AUC**ₗₐₛₜ is the area under the concentration-time curve from the moment of drug administration to the time of the last quantifiable concentration. | | | |

The clinical trial in healthy volunteers of a drug in the form of capsules containing excipients and 10 mg of MMSCs of formula Ib with an average particle diameter of 100 µm at doses of 10, 20, 30, 40 and 80 mg/day showed that the half-life of the Ib drug varied from 0.5 h to 3 h at doses of 10 and 20 mg (CV>70%) and from 1 h to 3 h at a dose of 30 mg (CV=35%). With constant administration of the drug, the concentration of the active metabolite Ia in the plasma increased reaching a maximum on average after 9.8 days of dosing at 10 mg/day; 7.5 days, at 20 mg/day; and 6.7 days, at 30 mg mg/day. The plasma half-life of metabolite Ia averaged 7.5 days at doses of 20 and 30 mg/day and 8.9 days at a dose of 10 mg/day.

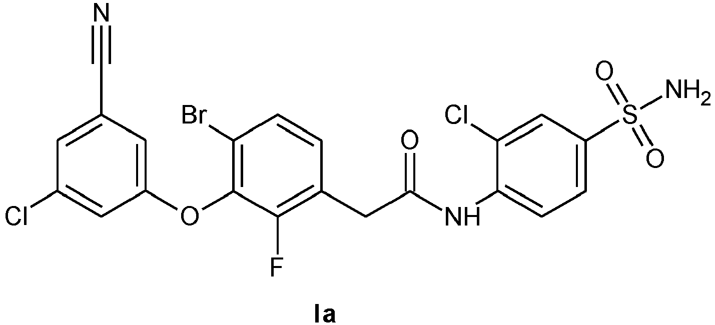

With multiple drug administration at a dose of 10 mg/day, Cₘₐₓ = 5.5 ng / ml (day 1) and Cmax = 75 ng/ml (day 14) of the active metabolite Ia in plasma and Cₘₐₓ = 825 ng/ml (day 14) in blood cells. With repeated administration at a dose of 20 mg/day, Cmax = 12.7 ng/ml (day 1) and Cₘₐₓ = 164 ng/ml (day 14) of the active metabolite Ia in plasma and Cₘₐₓ = 1041 ng/ml (day 14) in blood cells. With repeated administration of the drug at a dose of 80 mg / day, Cₘₐₓ = 250 ng/ml of the active metabolite of formula Ia in plasma and the half-life T1/2 = 122 hours.

With repeated weekly intake of a drug containing MMSCs of formula Ib with an average particle diameter of 100 µm by healthy volunteers at a dose of 41.4 mg/week, 82.8 mg/week, 124.2 mg/week, the maximum concentration of the active metabolite Ia in the first week is reached approximately 5.5 hours after taking the drug and has Cₘₐₓ values of 34.5 ng/ml, 63.6 ng/ml and 117.1 ng/ml, respectively, and a week later (168 hours), concentration values for the active metabolite Ia are 26.1 ng/ml, 36.9 ng/ml and 57.2 ng/ml, respectively.

A study of the efficacy, safety, and optimal dosage of MMSCs of formula Ib in HIV-1-infected patients who had not previously received treatment was carried out. The study used capsules containing 10 mg of the micronized monoclinic form VM-1500 with an average particle diameter of 100 µm. Doses of 20 mg/day (60 patients) and 40 mg/day (60 patients) of MMSCs of formula Ib were studied versus 600 mg/day of Efavirenz (30 patients). It was found that the MMSCs of formula Ib therapy groups did not differ in effectiveness (p=0.804) and were similar in effectiveness to the Efavirenz group (p=0.714). The drug containing the MMSCs of formula Ib in doses of 20 and 40 mg was not inferior to Efavirenz in terms of effectiveness in reducing the viral load to <400 copies/ml over a period of 12 weeks. There was a tendency for a statistical difference in the occurrence of a viral load reduction of 10 times and more (p=0.053) between the therapy groups treated with MMSCs of formula Ib, 20 mg, and Efavirenz, 600 mg. The therapy groups did not statistically differ in the frequency of achieving an undetectable HIV-1 RNA level at week 36 (p=0.408). The advantage of the MMSCs of formula Ib 20 mg group over the Efavirenz 600 mg group was statistically significant (p=0.0237).

No development of HIV-1 resistance was established when using MMSCs of formula Ib at a dose of 20 mg or Efavirenz at a dose of 40 mg. No serious adverse events were observed with the use of MMSCs of formula Ib in patients.

Another subject of the present invention is the use of a pharmaceutical composition of this invention for the prevention and treatment of human immunodeficiency virus (HIV) and acquired immunodeficiency syndrome (AIDS).

A further subject of this invention is the use of a medicinal product of this invention for the prevention and treatment of human immunodeficiency virus (HIV) and acquired immunodeficiency syndrome (AIDS).

Yet another subject of this invention is a method for treating a HIV-infected patient by oral administration of a pharmaceutical composition or a medicinal product of this invention.

A still further subject of this invention is a method of treating a patient by oral administration once a day of a drug of this invention containing from 20 mg to 41.4 mg MMSCs of formula Ib.

A still further subject of this invention is a method of treating a patient by oral administration once a week of a drug of this invention containing from 41.4 mg to 207 mg MMSCs of formula Ib.

Methods for preventing and treating HIV infection in subjects disclosed herein include administration of a new oral dosage form disclosed herein (in particular, a capsule) to a subject, usually human, typically once a day.

In some embodiments, the methods disclosed herein include repeated injections once a day. For example, in some embodiments, the methods disclosed herein include administration of new oral dosage forms disclosed herein daily or once a week.

The subject of this invention is also a method for preventing HIV infection in subjects at risk of infection by administering a pharmaceutical composition or a drug of this invention.

The subject of this invention is also a method for the prevention of HIV infection in subjects at risk of infection by one-time administration of a drug (a pharmaceutical composition or a medicinal product) of this invention.

In some embodiments, the methods disclosed herein include administration before and/or after an event that could expose a person to HIV or that would otherwise increase a person's risk of HIV infection, for example, as pre-exposure prophylaxis and/or as post-exposure prophylaxis. Examples of events that could increase a person's risk of HIV infection include, without limitation, condom use during anal intercourse with an HIV-positive partner or a partner of unknown HIV status; anal sex with more than 3 sexual partners; sex with a male partner and diagnosis of sexually transmitted infections; and no consistent condom use with a sexual partner who is known to be HIV-positive.

In some embodiments, for example, when administered for prophylactic purposes, the new solid oral dosage forms disclosed herein are administered from 2 to 72 hours, from 2 to 48 hours, from 2 to 24 hours, or from 2 to 12 hours before an event that increases a person's risk (for example, before sex or other HIV infection). In some embodiments, the solid oral dosage forms disclosed herein are administered within 72 hours, 60 hours, 48 hours, 24 hours, 12 hours, 9 hours, 6 hours, 4 hours, 3 hours, 2 hours, or 1 hour before an event that will increase the risk of HIV infection (for example, prior to a sexual intercourse or other exposure to HIV infection ).

In some embodiments, the new oral solid dosage forms disclosed herein are administered prior to an event that would increase the risk of human exposure to HIV, they are administered daily prior to the event. In some embodiments, when the solid oral dosage forms disclosed herein are administered prior to an event that would increase the risk of human exposure to HIV, they are administered one to three times prior to the event.

In some embodiments, for example, when administered as part of a prevention regimen, the new solid oral dosage forms disclosed herein are administered 2 to 48 hours, 2 to 36 hours, 2 to 24 hours, or 2 to 12 hours after an event that will increase the risk of HIV infection (for example, after a sexual intercourse or other exposure to HIV infection).

In some embodiments, for example, in prophylaxis, the new solid oral dosage forms disclosed herein are administered within 7 days, 14 days, 21 days, 28 days, 30 days, or 45 days after an event that increases an individual's risk of acquiring HIV (for example, after a sexual intercourse or other exposure to HIV).

In some embodiments, such as in prevention, the new solid oral dosage forms disclosed herein are administered within 30 days of an event that increases an individual's risk of HIV infection (for example, after sex or other HIV exposure). In some embodiments, the solid oral dosage forms described herein are administered less than 1 hour, 2 hours, 3 hours, 4 hours, 5 hours, 6 hours, 7 hours, 8 hours, 9 hours, 12 hours, 18 hours, 24 hours, 36 hours, or 48 hours after an event that would increase a person's risk of infection (for example, after a sexual intercourse or other exposure to HIV). In some other embodiments, the solid oral dosage forms disclosed herein are administered within 1 day, 2 days, 3 days, 4 days, or 5 days after an event that would increase a person's risk of infection (for example, after a sexual intercourse or other exposure to HIV). In some embodiments, when the new solid oral dosage forms disclosed herein are administered after an event that would increase a person's risk of HIV infection, they are prescribed daily. In some embodiments, when the new solid oral dosage forms disclosed herein are administered after an event that increases a person's risk of HIV infection, they are administered one to three times after the event. In some embodiments, when the solid oral dosage forms disclosed herein are administered after an event that increases a person's risk of HIV infection, they are administered once after the event.

The present invention will be described further in connection with certain embodiments that are not intended to limit the scope of the invention. On the contrary, the present invention covers all alternatives, modifications and equivalents that may be included in the scope of the claims. Thus, the following examples, which include specific embodiments, illustrate, but do not limit, the present invention.

The invention is illustrated by the following drawings:
Figure 1. Diffractogram of the crystal sample of compound 1b.
Figure 2. Simulation of the diffractogram of a crystal sample of compound 1b by the Pauli method. The blue line indicates the experimental diffractogram (1); the red line, the calculated one (2); and the gray line, the difference curve (3).
Figure 3. DSC curve of monoclinic symmetry crystals of compound 1b.
Figure 4. Thermogram curve of monoclinic symmetry crystals of compound 1b.

### THE BEST EMBODIMENT OF THE INVENTION

### Example 1. Micronized crystals of monoclinic symmetry sodium {[4-({[4-bromo-3-(3-chloro-5-cyanophenoxy)-2-fluorophenyl]acetyl}amino)-3-chlorophenyl]-sulfonyl}(propanoyl)azanide (MMSCs of formula Ib).

Compound Ib (3.0 kg) is dissolved in a mixture of isopropanol (14 1) and water (1 liter) at 60-70°C. The resulting solution is filtered hot through a layer of celite. n-Butyl acetate (30 l) is added to the filtrate, the solution is heated to a boil, and light-boiling components (30±21) are distilled from the mixture at 400±50 mm Hg. A suspension is formed, and the temperature of the cubic residue rises to 80° C; the vacuum is then removed, the resulting suspension is heated to 100-110°C and filtered. The sediment on the filter is filtered out and washed on the filter with 10 liters of n-butyl acetate heated to 60±5°C. The resulting product is dried 24 hours in air and then 24 hours at 45°C and at a residual pressure of 50±10 mbar in a drying cabinet to obtain 2.82±0.1 kg (94%) MMSCs of formula Ib with an average particle diameter of 95-130 µm and mp 247.67°C (Figure 3), said product being, according to XRD, crystals of monoclinic symmetry (Figures 1 and 2, Table 1). Unit cell parameters: crystallographic group P21/c, unit cell volume 2623.79 (12) Å3. Lattice parameters: a = 15.0637 (4) Å, b = 25.2592 (6) Å, c = 6.89727(19) Å, β =91.216(2)° obtained after refining diffractograms by the Pauli method.

MMSCs of formula Ib with an average particle diameter of 40 µm were obtained by additional grinding of MMSCs of formula Ib with an average particle diameter of 95-130 µm using an ultracentric ZM 200 mill (Retsch GmbH, Germany), and MMSCs of formula Ib with an average particle diameter of 10 µm were obtained by additional grinding of MMSCs of formula Ib with an average particle diameter of 95-130 µm using an ALPINE Aeroplex-100 AS spiral jet mill (Hosokawa Micron Corporation, Japan).

### Example 2. X-ray diffraction analysis of crystal samples of compound Ib.

XRD data of the samples were taken using a Bruker D8 Advance Vario powder X-ray diffractometer equipped with a copper anode X-ray tube and a Ge(III) monochromator (CuKα1) and a position-sensitive LynxEye detector, in transmission mode. The survey interval was 4.5-60° for all samples, with a step of 0.01° 2θ. The diffractograms were described in the Bruker Topas 5 software package-inode X-ray tube and a Ge(III) monochromator (CuKa1) and a position-sensitive LynxEye detector, in lumen installations. The survey interval was 4.5-60° for all samples, with a step of 0.01° 2θ. The diffractograms were described in the Bruker Topas 5 software package.

It was found that peaks observed on the diffractograms of the samples (Table 1, Figures 1 and 2, Table 2).1) had positions corresponding to the monoclinic space group P21/c with the following unit cell parameters: *a* = 15.0637(4)Å, *b* = 25.2592(6)Å, c = 6.89727 (19)Å, β = 91.216(2)°, and volume 2623.79 (12)Å³.

### Example 3. Differential scanning calorimetry (DSC) of crystal samples of the monoclinic form of compound Ib.

DSC was carried out on the DSC 204 F1 Phoenix device (NETZSCH, Germany) according to the following program: 3 heats from 20 to 200°C at a speed of 10 K/min in argon current. The duration of one program is 3 hours and 7 minutes (Total time on the screen). The total running time of the device (with allowance made for the baseline) is 6 hours and 14 minutes. On the DSC curve of the sample (Figure 3), there is a melting peak at ~250°C (the beginning of the process is 244°C, the end of the process is 253.5°C), the thermal melting effect is 102.9 J/g.

### Example 4. Thermogravimetric analysis of crystal samples of the monoclinic form of compound Ib.

The analysis was carried out on the TG 209 F1 Libra instrument (NETZSCH, Germany) according to the following program: heating from 30 to 550°C at a speed of 10 K/min in argon current (Figure 4). The onset decomposition temperatureof the sample was 294.7°C, the total mass loss was 52.9% (taking into account the error of the instrument). The duration of one program is 1 hour 43 minutes (Total time on the screen).

### Example 5. Pharmaceutical composition and medication in the form of a solid gelatin capsule.

To prepare a pharmaceutical composition, all components are pre-sieved and povidone-K30 (0.214 kg) is placed in a container with purified water (0.856 kg) to obtain a humidifier (1.070 kg). MMSCs of formula Ib with an average particle diameter of 10 to 130 µm (0.453 kg), lactose monohydrate (7.367 kg), and croscarmellose sodium (0.274 kg) are mixed in a DIOSNA V 25 mixer. To the resulting mixture, the previously prepared humidifier solution (1.070 kg) was added while stirring. The resulting granulate is dried in a fluidized bed dryer DIOSNA MIDI LAB XP. The dry granulate is sifted on a vibrating screen GR 50, the sifted granulate is crushed on a rotary impact mill SR300. Depending on the amount of granulate obtained, the amount of croscarmellose sodium and magnesium stearate is calculated (if there are no losses during the granulate production operation, the amount of croscarmellose sodium is 2.073 kg and magnesium stearate, 0.753 kg). The resulting granulate is placed in a mixer RM 100/200 and stirred for 20 minutes, then croscarmellose sodium is added and the mixture is stirred for 5 minutes. Magnesium stearate is then added and the mixture is stirred for another one minite to yield a pharmaceutical composition for encapsulation in the form of white or almost white powder containing from 18.50 mg to 21.50 mg MMSCs of formula Ib calculated on an average capsule fill weight basis. The resulting composition is encapsulated on an AFT-Lab ACG capsule machine in solid gelatin capsules No. 1 with a white body and a red cap. The resulting capsules contain 20.7 mg of MMSCs of formula Ib in combination with 337.196 mg of lactose monohydrate (Lactose 200 M), 21.168 mg of croscarmellose sodium (Vivasol), 9,800 mg of povidone-K30 (Povidone, Kollidon 30), and 3.136 mg of magnesium stearate (Magnesium stearate ST-V). The average weight of capsule fill is 392 mg (varying from 362.6 mg to 421.4 mg) and the average weight of filled capsules is 468 mg (varying from 434.90 mg to 503.10 mg).

Similarly, capsules containing 10 mg, 41.4 mg, 82.8 mg and 103.5 mg of MMSCs of formula Ib with an average particle diameter of 10 to 130 µm and capsules containing MMSCs of formula Ib with an average particle diameter of more than 130 µm are obtained.

### Example 6. Pharmacokinetic studies of finished dosage forms in capsules containing MMSCs of formula Ib with an average particle diameter of 100-200 µm in Beagle dogs.

The study was conducted on nine 7-8-month-old male Beagle dogs weighing 12.5-15.2 kg in three experimental groups of 3 dogs per group. Before the administration of capsules containing 20.7 mg of MMSCs of formula Ib, the dogs were deprived of food for at least 8 hours and water for at least 1 hour. Upon administering the drugs, liberal water intake was allowed after 2 hours and feeding after 10 hours. Blood plasma was collected before administration of the drug (0) and 15, 30 minutes, 1, 1.5, 2, 3, 4, 6, 8, 10, 12, 24, 36, 48, 72, 96, and 168 hours after administration. The volume of blood taken from one dog at one timepoint was no more than 1 ml and no more than 18 ml per week in total, which did not exceed the recommended 7.5% (64 ml) of the total blood volume in dogs.

Blood was collected in a volume of 1 ml at each timepoint in each animal from the lateral saphenous vein of the lower leg into test tubes containing a 0.5 M EDTA anticoagulant (100 µl). The plasma was separated by blood centrifugation at 10,000 rpm for 10 minutes, transferred to pre-labeled Eppendorf tubes, and frozen at -70°C prior to analysis. Blood plasma samples were stored on dry ice.

The analysis of blood plasma samples was performed by HPLC-MS/MS method with a lower limit of quantitative determination of the active metabolite Ia (0.5 ng/ml).

The main pharmacokinetic characteristics of capsules containing MMSCs of formula Ib in the form of micronized granules with an average particle diameter of 100 µm (group 1), 130 µm (group 2), and 200 µm (group 3) are presented in Table 2.

### Example 7. Pharmacokinetic studies of finished dosage forms in healthy volunteers.

The pharmacokinetics of finished dosage forms was studied in healthy volunteers in the same way as described above in example 5. The clinical trial of a drug in the form of a capsule (Example 4) containing excipients and 10 mg of MMSCs of formula Ib with an average particle diameter of 100 µm at doses of 10, 20, 30, 40, and 80 mg/day revealed that the half-life of the Ib prodrug varied from 0.5 h to 3 h at doses of 10 and 20 mg (CV>70%) and from 1 h to 3 h at a dose of 30 mg (CV=35%). In contrast to prodrug Ib, the active metabolite Ia showed accumulation in the blood after repeated drug administration as well as a long half-life. The concentration-time pattern of metabolite Ia in the blood was evaluated by its content in the plasma and blood cells. It was found that the concentration of the metabolite in the blood cells was an order of magnitude higher than in the plasma.

With constant administration of the drug, the concentration of the active metabolite Ia in the plasma increased reaching a maximum after an average of 9.8 days of dosing at a dose of 10 mg/day; 7.5 days, at a dose of 20 mg/day; and 6.7 days, at a dose of 30 mg/day. The plasma half-life of metabolite Ia averaged 7.5 days at doses of 30 and 20 mg/day and 8.9 days at a dose of 10 mg/day.

With repeated administration of the drug at a dose of 10 mg/day, Cₘₐₓ = 5.5 ng/ml (day 1) and Cₘₐₓ = 75 ng/ml (day 14) of the active metabolite Ia in plasma and Cₘₐₓ = 825 ng/ml (day 14) in blood cells. With repeated administration of the drug at a dose of 20 mg/day, Cₘₐₓ = 12.7 ng/ml (day 1) and Cₘₐₓ = 164 ng/ml (day 14) of the active metabolite Ia in the plasma and Cₘₐₓ = 1041 ng/ml (day 14) in blood cells. With repeated administration of the drug at a dose of 80 mg/day, Cₘₐₓ = 250 ng/ml of the active metabolite Ia in plasma and the half-life T1/2 = 122 hours.

With repeated weekly administration of the drug containing MMSCs of formula Ib with an average particle diameter of 100 µm in healthy volunteers at doses of 41.4 mg/week, 82.8 mg/week, or 124.2 mg/week, the maximum concentration of active metabolite Ia in the first week is reached approximately 5.5 hours after taking the drug and has a value of 34.5 ng/ml, 63.6 ng/ml and 117.1 ng/ml, respectively; a week later, the concentration of active metabolite Ia is 26.1 ng/ml, 36.9 ng/ml. and 57.2 ng/ml, respectively.

### Example 8. Evaluation of the efficacy of finished dosage forms in HIV-infected patients.

Evaluation of the efficacy, safety, and optimal dosage of MMSCs of formula Ib in HIV-1-infected patients who have not previously received treatment. The study used capsules (same as in Example 4), excipients and 10 mg of MMSCs of formula Ib with an average particle diameter of 100 µm. Doses of 20.7 mg/day (60 patients) and 41.4 mg/day (60 patients) of MMSCs of formula Ib were studied in comparison with Efavirenz (600 mg/day, 30 patients). It was found that the MMSCs of formula Ib therapy groups did not differ in efficacy (p=0.804) and were similar in efficacy to the Efavirenz group (p=0.714). The drug with the MMSCs of formula Ib at doses of 20 and 40 mg was not inferior to Efavirenz in reducing the viral load to < 400 copies/ml over a period of 12 weeks. At week 12, the viral load decreased by -2.8±0.7 in the group receiving 20 mg MMSCs of formula Ib and by -2.7±1.0 in the Efavirenz group receiving 600 mg. At week 24, the changes were already - 3.3±0.7 and -3.3±0.8, respectively. There was a tendency for a statistical difference in the occurrence of a viral load reduction of 10 times and more (p=0.053) between the therapy groups treated with MMSCs of formula Ib, 20 mg, and Efavirenz, 600 mg. These therapy groups did not differ statistically in the frequency of achieving undetectable HIV-1 RNA levels at week 36 (p=0.408). The advantage of the 20 mg MMSC group over the 600 mg Efavirenz group was statistically significant (p=0.0237).

The development of HIV-1 resistance while using MMSCs of formula Ib and Efavirenz at the studied doses has not been established. No serious adverse events were observed in using MMSCs of formula Ib in patients.

### INDUSTRIAL APPLICABILITY

The invention can be used in human medicine and veterinary medicine.

## Claims

1. The active ingredient of a pharmaceutical composition consisting of micronized monoclinic symmetry crystals (MMSCs) of sodium {[4-({[4-bromo-3-(3-chloro-5-cyanophenoxy)-2-fluorophenyl]acetyl}amino)-3-chlorophenyl]-sulfonyl}(propanoyl)azanide of formula Ib 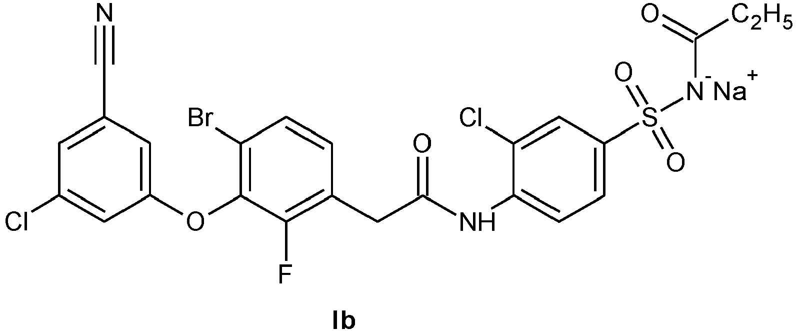 with an average particle diameter ≤130 µm.

2. An antiviral pharmaceutical composition containing MMSCs of formula Ib according to claim 1 in combination with pharmaceutically acceptable excipients.

3. The pharmaceutical composition according to claim 2 wherein the average particle diameter of MMSCs of formula Ib varies from 10 µm to 110 µm.

4. The pharmaceutical composition according to claims 2, 3 with excipients comprising at least lactose monohydrate, sodium croscarmellose, povidone, and magnesium stearate.

5. The pharmaceutical composition according to any of claims 2, 3, or 4 containing 10.0% MMSCs of formula Ib, 81.7% lactose monohydrate, 5.1% croscarmellose sodium, 2.4% povidone, and 0.8% magnesium stearate.

6. The pharmaceutical composition according to any of claims 2, 3, or 4 containing 15.1% MMSCs of formula Ib, 76.2% lactose monohydrate, 5.4% croscarmellose sodium, 2.5% povidone, and 0.8% magnesium stearate.

7. A medicinal product containing MMSCs of formula Ib according to claim 1 with an average particle diameter ≤130 µm in combination with pharmaceutically acceptable excipients.

8. The medicinal product according to claim 7 with excipients comprising at least lactose monohydrate, sodium croscarmellose, povidone, and magnesium stearate.

9. The medicinal product according to claims 7, 8 containing 41.4 mg MMSCs of formula Ib, 337.2 mg lactose monohydrate, 21.2 mg sodium croscarmellose, 9.8 mg povidone, and 3.1 mg magnesium stearate.

10. The medicinal product according to claims 7, 8 containing 20.7 mg MMSCs of formula Ib, 104.4 mg lactose monohydrate, 7.4 mg sodium croscarmellose, 3.4 mg povidone, and 1.1 mg magnesium stearate.

11. The medicinal product according to any of claims 7-10 with an average diameter of MMSCs of formula Ib varying from 10 µm to 110 µm.

12. A method for treating a HIV-infected patient by administering MMSCs of formula Ib according to claim 1, a pharmaceutical composition according to any of claims 2-6, or a medicinal product according to any of claims 7-11.

13. The method according to claim 12 for treating a patient by oral administration once a day of a drug containing from 20 mg to 41.4 mg MMSCs of formula Ib.

14. The method according to claim 13 for treating a patient by oral administration once a week of a drug containing from 41.4 mg to 207 mg MMSCs of formula Ib.

15. A method for the prevention of HIV infection in subjects at risk of infection, by administering MMSCs of formula Ib according to claim 1, a pharmaceutical composition according to any of claims 2-6, or a medicinal product according to any of claims 7-11.

16. The method according to claim 15 by one-time administration of a drug containing from 20 mg to 207 mg MMSCs of formula Ib.
